# EUROPEAN PATENT SPECIFICATION

(11) **EP 3 102 180 B1**
(45) Date of publication and mention of the grant of the patent: **03.04.2019**
(21) Application number: 15702750.9
(22) Date of filing: 03.02.2015
(51) Int. Cl.: A61K 8/49, A61K 8/43, A61K 8/34, A61K 8/19, A61Q 17/00, A61Q 19/00, A61Q 19/10, A61K 31/365, A61K 33/38

(54) **A TOPICAL COMPOSITION**
TOPISCHE ZUSAMMENSETZUNG
COMPOSITION TOPIQUE

(30) Priority: 07.02.2014 EP 14154330
(43) Date of publication of application: 14.12.2016
(73) Proprietor: Unilever N.V., 3013 AL Rotterdam (NL); Unilever PLC, a company registered in England and Wales under company no. 41424 of, London EC4Y 0DY (GB)
(72) Inventor: DASGUPTA, Anindya, Bangalore 560 066 (IN); KHOKHAR, Jasmeet Kaur, Bangalore 560 066 (IN); MAJUMDER, Suman, Bangalore 560 066 (IN)
(74) Representative: van den Brom, Coenraad Richard
(86) International application number: PCT/EP2015/052191
(87) International publication number: WO 2015/117957

(56) References cited:
- WO-A1-01/43704
- WO-A1-02/09698
- CN-A- 1 446 541
- DE-A1- 10 341 179
- FR-A1- 2 877 218
- US-A1- 2004 156 873
- US-A1- 2006 009 499

## Description

### Field of the invention

The invention relates to a composition for external application for care of skin, oral cavity, scalp or hair. The invention more particularly relates to a composition for treating acne and is efficacious even when formulated as a wash-off composition.

### Background of the invention

The invention relates to a composition for care of skin, oral cavity, hair and scalp.
Acne, also known as Acne vulgaris, is a common skin condition that affects nearly all adolescents and adults at some times in their lives. It has a complex etiology, involving abnormal keratinization, excess sebum production, androgen function, bacterial growth, and immune hypersensitivity. Although one or more of the above processes is correlated with acne, the one triggering factor and the exact sequence of events leading to the formation of acne lesions has not been fully understood. Other factors which have been linked to acne are presence of free radicals with subsequent oxidative stress leading to cellular damage. It has been observed that acne usually occurs in areas rich in sebaceous glands like the face, neck and back. A bacteria *Propionibacterium acnes* (*P. acnes*) has also been implicated in occurrence of acne.

Acne has been treated in many ways. Most treatments take several weeks to months before a noticeable change is seen. Benzoyl peroxide which has an antibacterial effect has been used for mild cases of acne and is also believed to prevent formation of further acne. In very severe cases of acne, antibiotics like tetracycline, erythromycin and clindamycin have been used. Antibiotics are believed to work by several mechanisms, the most important being the decrease in the number of bacteria in and around the follicle. They are also thought to reduce the irritating chemicals produced by the white blood cells in the sebum, thereby reducing the inflammatory response.

Most of the treatments for acne, involve use of synthetic chemicals. Many people do not prefer use of synthetic chemicals since they are believed to be harsh on the human body. Some people believe that synthetic chemicals cause side effects. Hence, more and more people prefer use of materials which are "natural" e.g. actives based on herbal origin.

The present inventors have also been working towards providing such "natural" solutions to solving the problem of acne. The present inventors have found that a molecule andrographolide, present in extract of *Andrographis paniculata,* heretofore known to provide skin lightening and barrier protection to skin is a very potent anti-inflammatory agent. Further, they have found that a composition comprising this molecule along with select antimicrobial agents can be used to treat acne. Further when this material is used along with certain antimicrobials, excellent dentifrices and mouthwashes can be formulated.

Andrographolide has been used in compositions for external application.

CN102058502 (Tianjin Bijia, 2011) discloses a cleaning agent for an oral cavity, mainly comprising the following ingredients in percentage by weight: 0.1-30 percent of green tea, 0.1-50 percent of turmeric, 0.01-10 percent of menthyl anthranilate, and 0.01-10 percent of sodium cyclamate and acesulfame. According to different conditions of patients, 0.1-50 percent of scutellaria, 0.1-50 percent of camellia, 0.1-30 percent of andrographolide and 0.01-10 percent of borneol can also be added.

US2006263449 (Advanced Gene Technology, 2006) discloses a method for treating arthritic disorders, skin inflammatory disorders and pain, comprising administering to a subject an extract of Andrographis paniculata Nees, and also provides a herbal composition used therefor that comprises the extract of Andrographis paniculata Nees. The method is generally envisaged for ingestion and not for external application.

None of the published documents disclose a combination of andrographoide and an antimicrobial active to combat the problem of acne.

It is thus an object of the present invention to provide for a cosmetic composition to treat acne.

It is another object of the present invention to provide for a cosmetic wash off composition to treat acne that is effective in the short time frames that wash-off compositions are generally used for.

It is yet another object of the present invention to provide for a composition that provides enhanced care of the oral cavity, scalp and hair.

### Summary of the invention

According to the first aspect of the present invention there is provided a topical composition comprising
(i) a compound of the Formula 1, andrographolide; 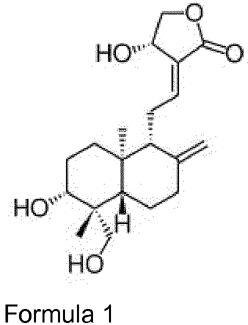
(ii) an antimicrobial agent; and
(iii) a cosmetically acceptable base;
wherein said antimicrobial agent is a combination of thymol and terpineol.

It is particularly preferred that the composition according the invention is a composition for use in the treatment of acne.

According to another aspect of the present invention there is provided a composition for use in a method of cleansing the external surface of the body comprising the steps of
(i) applying a composition of the first aspect on to the desired external surface; and optionally
(ii) at least partially removing the composition from the surface.

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated.

Except in the operating and comparative examples, or where otherwise explicitly indicated, all numbers in this description indicating amounts of material or conditions of reaction, physical properties of materials and/or use are to be understood as modified by the word "about". Unless specified otherwise, numerical ranges expressed in the format "from x to y" are understood to include x and y. When for a specific feature multiple preferred ranges are described in the format "from x to y", it is understood that all ranges combining the different endpoints are also contemplated.

By a topical composition is meant a composition for external application in the form of a leave-on or wash-off format meant for cleaning or disinfecting topical areas e.g. skin and/or hair and or oral cavity of mammals, especially humans. Such a composition includes any product applied to a human body for also improving appearance, cleansing, odor control or general aesthetics. It is more preferably a rinse off product. The composition of the present invention may be in the form of a liquid, lotion, cream, foam or gel, or toner, or applied with an implement or via a face mask, pad or patch. "Skin" as used herein is meant to include skin on the face and body (e.g., neck, chest, back, arms, underarms, hands, legs, buttocks and scalp). The composition of the invention is also of relevance to applications on any other external substrates of the human body other than skin e.g. hair and oral cavity.

The present invention relates to a composition for external application comprising andrographolide, an antimicrobial agent and a cosmetically acceptable base. Andrographolide has the structure

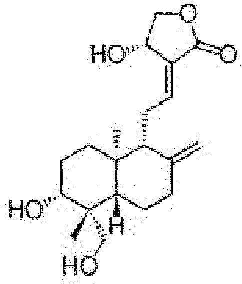

Andrographolide is preferably included as an extract of *Andrographis paniculata.*

*Andrographis paniculata Nees* has been used for medicinal purposes in traditional medicine in India, China and Thailand. In Thailand, it has long been promoted in primary health care as a cure for sore throat. The main ingredients are diterpenic lactones, including for example andrographolide, 14-deoxyandrographolide, 14-deoxy-11, 12-didehydroandrographolide, and neoandrographolide. Andrographolide and its derivatives are considered to be the main active compounds with immunostimulant, antipyretic and anti-diarrhoeal properties. The leaves of *Andrographis paniculata,* popular in Scandinavia as a cold and influenza remedy, are used traditionally in ayurvedic, thai and Chinese medicine to treat fever associated with infectious diseases.

*A. paniculata,* known on the Indian subcontinent as Chirayetah and Kalmegh in Urdu and Hindi languages, respectively, is an annual plant, 1-3 ft high, that has been used in traditional systems of Unani and Ayurvedic medicines. It is called Creat in English and is known as the "king of bitters." It grows in hedge rows throughout the plains of India and is also cultivated in gardens. It also grows in many other Asian countries and is used as a traditional herbal medicine in China, the Philippines, Malaysia, Indonesia, and Thailand

The composition preferably comprises 0.001 to 5%, preferably 0.005 to 5%, further more preferably 0.01 to 2% andrographolide, by weight of the composition.

The composition of the invention also comprises an antimicrobial agent. The antimicrobial agent is a combination of thymol and terpineol.

When a silver compound is used a the antimicrobial active, the preferred silver compounds are silver nitrate, silver halide, silver hydroxide or silver oxide.

Cationic compound are another preferred class of antimicrobial actives for use in the present invention. Preferred cationic compounds are benzalkonium chloride, cetyl pyridinium chloride or cetyl trimethyl ammonium bromide.

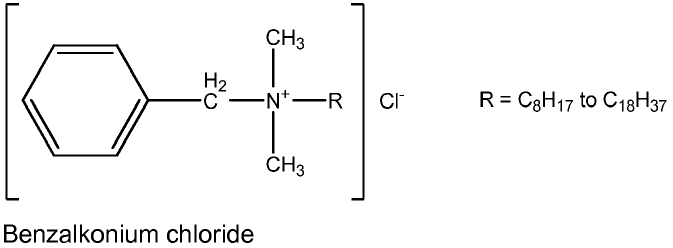

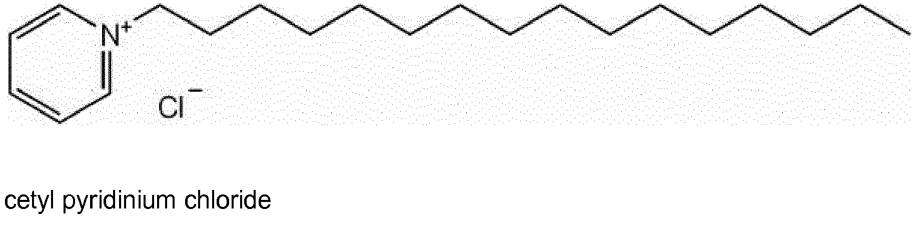

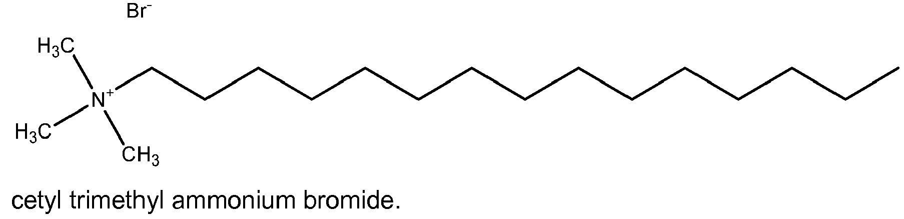

Essential oil actives are yet another highly preferred antimicrobial actives for use in the composition of the present invention. Preferred essential oil actives are thymol, eugenol, menthol, geraniol, vertenone, eucalyptol, pinocarvone, dedrol, anethol, carvacrol, hinokitiol, berberine, ferulic acid, cinnamic acid, methyl salicylate, terpineol or limonene. More preferred essential oil actives are

The structures of thymol and terpineol are given below:

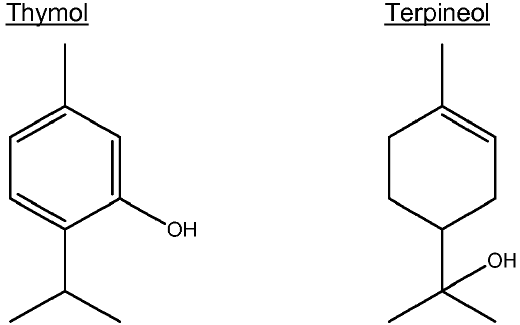

The composition of the invention preferably comprises 0.0001 to 5%, more preferably 0.001 to 2%, further more preferably 0.01 to 1% antimicrobial agent.

Without wishing to be bound by theory, the inventors believe that the ingredients of the composition of the invention interact synergistically by way of the following mechanism. The antimicrobial agent reduces the microbial load so that number of micro-organism (*P.acne*) binding to TLR-2 receptor of a cell are reduced resulting in decreased inflammation. Pre-treatment of cells with the Andrographolide leads to inhibition of NF-kappa B translocation which further leads to decreased expression of inflammatory markers. Combination of Antimicrobial agent and Andrographolide would be helpful in reducing the inflammation as the trigger (*P.acne*) is reduced by an anti-microbial agent and the effect (expression of inflammatory markers) produced due to reduced trigger is furthermore suppressed by an anti-inflammatory agent.

The composition of the invention is formulated such that the cosmetically acceptable base is preferably in the form of a cream, lotion, face wash, gel, emulsion, dentifrice, mouthwash, shampoo, hair oil or conditioner.

The composition of the invention is preferably a wash-off composition and this is enabled by including 1 to 80% by weight of a surfactant.

In general, the surfactants may be chosen from the surfactants described in well known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981. Any type of surfactant, i.e. anionic, cationic, nonionic, zwitterionic or amphoteric can be used.

A particularly preferred surfactant is soap. Soap is a suitable surfactant for personal washing applications of the composition of the invention. The soap is preferably C8-C24 soap, more preferably C10-C20 soap and most preferably C12-C16 soap. The soap may or may not have one or more carbon-carbon double bond or triple bond. The cation of the soap may be alkali metal, alkaline earth metal or ammonium. Preferably, the cation of the soap is selected from sodium, potassium or ammonium. More preferably the cation of the soap is sodium or potassium.

The soap may be obtained by saponifying a fat and/or a fatty acid. The fats or oils generally used in soap manufacture may be such as tallow, tallow stearines, palm oil, palm stearines, soya bean oil, fish oil, castor oil, rice bran oil, sunflower oil, coconut oil, babassu oil, palm kernel oil, and others. In the above process the fatty acids are derived from oils/fats selected from coconut, rice bran, groundnut, tallow, palm, palm kernel, cotton seed, soyabean, castor etc.

A typical fatty acid blend consisted of 5 to 30% coconut fatty acids and 70 to 95% fatty acids ex hardened rice bran oil. Fatty acids derived from other suitable oils/fats such as groundnut, soybean, tallow, palm, palm kernel, etc. may also be used in other desired proportions. The most preferred soap is a laurate soap. The soap, when present in solid forms of the present invention is present in an amount of 30 to 90%, preferably from 50 to 85%, more preferably 55 to 75% by weight of the composition. The soap, when present in liquid forms of the composition is present in 0.5 to 20%, preferably from 1 to 10% by weight of the composition.

Alternately the surfactants are non-ionic surfactants, such as C8-C22, preferably C8-C16 fatty alcohol ethoxylates, comprising between 1 and 8 ethylene oxide. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Other anionic surfactants preferred are primary alkyl sulphates, secondary alkyl sulphonates, alkyl benzene sulphonates, or ethoxylated alkyl sulphates. Alkyl polyglucoside may also be present in the composition, preferably those having a carbon chain length between C6 and C16. Suitable surfactant concentrations in liquid forms of cleaning application are generally more than 0.5 but less than 10%, preferably from 1 to 5 % by weight of the composition. In solid compositions, the surfactant is preferably present in 5 to 40%, preferably from 10 to 30% by weight of the composition.

The antimicrobial composition of the invention is useful in oral care compositions e.g. in a dentifrice/ toothpaste or oral rinse product. In such applications, preferred surfactants are anionic, non-ionic or amphoteric in nature, preferably anionic or amphoteric. The Anionic surfactant is preferably an alkali metal alkyl sulphate, more preferably a sodium lauryl sulphate (SLS) . Mixtures of anionic surfactants may also be employed. The amphoteric surfactant is preferably a betaine, more preferably an alkylamidopropyl betaine (wherein the alkyl group is a linear C10∼C18 chain), and most preferably is cocoamidopropyl betaine (CAPB). Mixtures of amphoteric surfactants may also be employed. Suitable surfactant concentrations in oral care application are generally from about 2% to about 15%, preferably from about 2.2% to about 10%, more preferably from about 2.5 to about 5% by weight of the total composition.

Thus, in a highly preferred aspect, the antimicrobial composition includes soap, alkyl sulphate or linear alkyl benzene sulphonate as the surfactants.

The method of the invention is made effective by formulating a composition of the invention in a suitable carrier. Water is a preferred carrier. Water may preferably be present in 10 to 90% by weight of the composition depending on the format of the composition. In solid compositions, water may be present in 10-30%, while in liquid or semi-solid composition, water may be present in 40 to 90%.

The composition of the invention is especially suitable for use in a wash off process where the contact time of the antimicrobial actives with the surface is low, i.e of the order of less than 5 minutes, preferably less than 2 minutes, further more preferably less than a minute and in many cases less than 15 seconds.

According to another aspect of the present invention there is provided a composition for use in a method of cleansing the external surface of the body comprising the steps of
(i) applying a composition of the invention on to the desired external surface; and
(ii) optionally at least partially removing the composition from the surface.

According to a preferred aspect the composition is substantially completely removed from the surface. Further more preferably it is rinsed from the surface with a suitable solvent or wiped from the surface with a suitable wipe.

The solvent for rinsing the surface is preferably water but could also be a mixture of water and alcohol. The word rinsing herein includes the act of wiping the surface with a suitable wipe. Thus the surface e.g hand, face, body, oral cavity is first contacted with the composition of the invention. It is then rinsed preferably with sufficient amounts of water after a pre-determined period of time to remove any visible or sensory reside of the composition. Alternately an alcohol wipe or a water/alcohol impregnated wipe may be used to wipe the surface to be visibly free of the composition. The step of rinsing the substrate is preferably carried out less than 5 minutes, preferably less than 2 minutes, further more preferably less than a minute and in many cases less than 15 seconds after the step of applying the composition on the substrate.

Yet another aspect of the present invention relates to a composition of the invention for use in the treatment of acne.

The invention will now be illustrated with the help of the following non-limiting examples.

### Examples

### Examples A. B and 1: Anti-inflammatory potential of andrographolide (Example -1) as compared to control (Example - A) and a positive Control simvastatin (Example - B).

The procedure used to measure the anti-inflammatory potential of the various samples was as follows:
Blood was diluted with equal volume of HBSS or PBS(1:1). The PBMCs were isolated into fresh tube. These PBMC's were then plated in 96 well plate (10⁶ cell/well). They were then pretreated with the active (for 24 hours). Induction was then carried out with antigen/mitogen for 24 hours. The cell-supernatant was then taken and frozen. The cytokine levels in these supernatants were detected by ELISA.

The positive control Simvastatin has the structure:

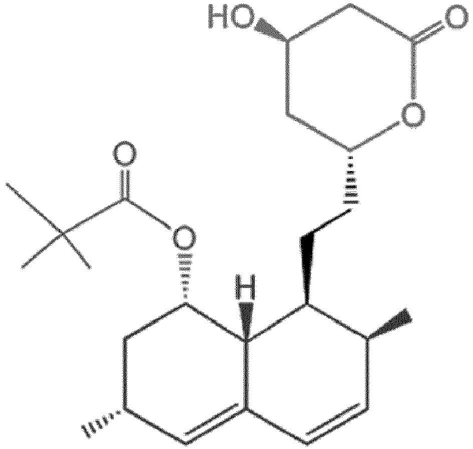

The IL-17 concentration (pg/ml) was measured and the data is presented in Table -1 for the following three cases:
Example - A: Control: 25 ng PMA/ 1 µg lonomycin.
Example - B: 0.001% Simvastatin
Example - 1: 0.001% Andrographolide.

**Table - 1**

| Example | Active | IL-17 concentration (pg/ ml) |
|---|---|---|
| A | Control = 25 ng PMA/ 1 µg lonomycin | 732 |
| B | 0.001% Simvastatin | 493 |
| 1 | 0.001% Andrographolide | 143 |

The lower the value of the IL-17 concentration, the higher is the potential as an anti-inflammatory active. Table -1 indicates that andrographolide is a highly potent anti-inflammatory agent.

### Example B and 2: Anti-inflammatory potential measured using TNF-alpha ELISA

The experiments were conducted using a similar procedure as earlier except that TNF-alpha ELISA was measured as a marker. The data is presented in Table -2 below.

**Table -2**

| Example | Active | TNF-alpha concentration (pg/ ml) |
|---|---|---|
| C | Control = 25 ng PMA/ 1 µg lonomycin | 5384 |
| 2 | 0.001% Andrographolide | 58 |

The lower the value of TNF-alpha, the higher is the potential as an anti-inflammatory agent.

The data in Table - 2 above indicates that andrographolide is also implicated as a potent anti-inflammatory active using another marker technique.

### Examples C - F and Example 3: Co-treatment with different actives

The experiments were conducted by co-treating with different actives using TNF-alpha ELISA technique and the data is presented in Table - 3 below:

**Table - 3**

| Example | Active | IL-17 concentration (pg/ ml) |
|---|---|---|
| C | Control = 25 ng PMA/ 1 µg lonomycin | 2244 |
| D | 0.001% Retinoic acid | 2762 |
| E | 0.001% Retinol | 2006 |
| F | 0.001% Extract of sage | 2473 |
| 2 | 0.001% Andrographolide | 1187 |

The data in Table -3 above indicates that andrographolide performs vastly superior in the above test for anti-inflammatory properties as compared to some of the well known and highly efficacious agents.

### Example G and 3: Efficacy of antimicrobial agents as per the invention as compared to conventional agent Trichlorocarbanilide

Efficacy of two compositions as shown in Table - 4 were measured as follows: The O.D of the bacterial cells were adjusted such that it corresponds to 1X10⁸ cells of bacteria. 1ml of the bacterial culture was taken and added to 9 ml of the test solution (containing the antimicrobial active). The mixture was kept for the desired amount of time after which it was neutralized by adding 1 ml of mix to the 9 ml of D/E neutralizer. It was then serially diluted till 10⁵. The number of bacteria was then enumerated by using the pour plate method.

The log CFU/ml for the two compositions is summarized in Table -4.

**Table-4**

| Example | Example G | Example 3 |
|---|---|---|
| Composition | 0.2% TCC | 0.2% thymol + 0.5% terpineol |
| time | Log CFU/ml | Log CFU/ ml |
| 15 seconds | 7.1 | Complete kill |
| 30 seconds | 7.1 | Complete kill |
| 1 minute | 7.1 | Complete kill |
| 5 minutes | 7.1 | Complete kill |

The data in Table - 4 indicates that the antimicrobial agents as per the invention provides for vastly improved antibacterial activity as compared to a conventional active, at very short contact time.

### Examples H and 4: Anti-inflammatory potential of andrographolide (Example -4) as compared to control (Example - H) when keratinocytes were used as the model cells.

The procedure used to measure the anti-inflammatory potential of the various samples was as follows:
The primary keratinocytes were seeded in a 24 well plate (80000 cell/well). On attaining full confluency, the cells were pretreated with the active for 24 hours. Inflammation was induced either through using LPS or through use of live and heat treated *P. acnes* for 24 hours. The cell-supernatant was then taken and frozen. The cytokine levels in these supernatants were detected by ELISA.

The TNF-alpha concentration (pg/ml) was measured and the data is presented in Table -5.

**Table - 5**

| Example | Active | TNF-alpha concentration (pg/ ml) |
|---|---|---|
| H | Control = 25 µg LPS | 283 |
| 4 | 0.001% Andrographolide | 156 |

The data in Table -5 above indicates that andrographolide is a potent anti-inflammatory active as indicated by invitro models using keratinocyte cells. Examples I, J and 5 to 8: Anti-inflammatory potential of andrographolide when keratinocytes were used as the model cells with LPS and heat treated acne are the inflammatory inducers and IL-8 marker is studied.

The experiments as shown in Table - 6 were studies using keratinocyte cell lines and the IL-8 concentration was measured. The data is summarized in Table -6.

**Table - 6**

| Example | Active | IL-8 concentration (pg/ ml) |
|---|---|---|
| I | Control = 50 µg LPS | 649 |
| 5 | 0.01% Andrographolide | 229 |
| 6 | 0.1% Andrographolide | 78 |
| J | Control = heat killed *P. acnes* | 2238 |
| 7 | 0.01% Andrographolide | 1088 |
| 8 | 0.1% Andrographolide | 230 |

### Examples K, 9 and 10: Experiments similar to J, 7 and 8 except that live p.acne was used

The data is summarized in Table - 7.

**Table-7:**

| Example | Active | IL-8 concentration (pg/ ml) |
|---|---|---|
| K | Control = live *P. acnes* | 558 |
| 9 | 0.01% Andrographolide | 337 |
| 10 | 0.1% Andrographolide | 205 |

The data in Tables 6 and 7 indicate that andrographolide is a potent anti-inflammatory active when keratinocyte cell lines are used with various other types of inflammatory inducers.

### Examples L to N and 11: Effect of composition of the invention on IL-8 inflammatory markers:

Actives/ Compositions as shown in Table-8 were measured for effect on inflammatory marker IL-8 using the following procedure:
PBMCs were isolated as discussed before for Example-1. They were then plated in a 96-well plate. In some wells, cells were pre-treated with 0.001% Andrographolide for 24 hours and some wells were not treated with anything. P.acne culture was grown on CY agar medium in aneorobic chamber and then culture was set to the .005 O.D. Bacterial culture was treated with antimicrobial active combination of thymol (0.05%) and terpineol (0.05%) for 30 seconds. After the contact time is over, sample was neutralized in D/E neutralizer. The bacterial pellet was collected by centrifugation. The pellet was re-suspended in cell culture medium and bacterial culture was added to the cells (PBMC's) treated with andrographolide and also bacterial culture was added to the wells where there was no treatment with andrographolide. Live *P.acne* (not treated with the antimicrobial actives) was added to the wells where there was no treatment with andrographolide and also added to the wells treated with andrographolide. The treated cells were incubated in 37° C incubator for 24 hours. After 24 hours the supernatant was removed and ELISA was done for IL-8 marker.

The IL-8 concentration was measured and the data is summarized in Table -8.

**Table - 8**

| Example | Active | IL-8 concentration (pg/ ml) |
|---|---|---|
| L | *P. acnes* | 957 |
| M | Andrographolide + *P. acnes* | 400 |
| N | P.acne treated with the antimicrbial | 158 |
| 11 | Andrographolide + *P. acnes* treated with the antimicrobial | 18 |

### Example: Effect of different antimicrobial actives along with Andrographolide on P.acne as tested against IL-8 inflammatory markers:

The same procedure was followed as explained in the previous section for this experiment. The antimicrobials which were used for this experiment were thymol, terpineol, Triclosan (TCN) and Trichlorocarban (TCC). The used concentrations of these antimicrobials and the concentration of the IL-8 marker after the treatment are summarized in the following Table 9.

**Table - 9**

| Example | Active along with their concentration | IL-8 concentration (pg/ ml) |
|---|---|---|
| P | *P.acne* | 641.5 |
| Q | Andrographolide + *P.acne* | 276.48 |
| R | 0.05% Thymol + *P.acne* | 546.38 |
| S | 0.05% Terpineol + *P.acne* | 599.8 |
| 12 | Andrographolide + 0.05% Thymol + 0.05% Terpineol + *P.acne* | 14.48 |
| T | 0.2% TCC + *P.acne* | 626.48 |
| U | Andrographolide + 0.2% TCC + *P.acne* | 306.48 |
| V | 0.2% TCN + *P.acne* | 613.15 |
| W | Andrographolide + 0.2% TCN + *P.acne* | 293.15 |
| X | 0.2% Silver Oxide + *P.acne* | 599.8 |
| 13 | Andrographolide + 0.2% Silver Oxide + *P.acne* | 243.15 |

From the above table it is evident that a combination of Andrographolide and selected antimicrobials provides a synergistic action on *P.acne.* It can be easily seen from the above table that, while a combination of thymol and terpineol and Andrographolide (Example 12) provides a superior synergistic action on *P.acne,* the other combinations of TCC + Andrographolide (Example U) and TCN + andrographolide (Example W) do not provide a synergistic effect. A similar kind of synergistic effect can also been seen with silver oxide and Andrographolide.

### Examples Y to Z1 and 14: Effect of composition of the invention on TNF-alpha inflammatory markers:

Experiments Example Y to Z and 14 to 17 were carried out in the same way as in the previous section, yet the ELISA was done for TNF- alpha marker.

The TNF- alpha concentration was measured and the data is summarized in Table -10.

**Table - 10**

| Example | Active along with their concentration | TNF-alpha concentration (pg/ ml) |
|---|---|---|
| Y | *P.acne* | 635.6 |
| Y1 | Andrographolide + *P.acne* | 231.15 |
| Y2 | 0.05% Thymol + *P.acne* | 546.94 |
| 14 | Andrographolide + 0.05% Thymol + *P.acne* | 170.63 |
| Y3 | 0.05% Terpineol + *P.acne* | 657.47 |
| 15 | Andrographolide + 0.05% Terpineol + *P.acne* | 210.10 |
| 16 | Andrographolide + 0.05% Thymol + 0.05% Terpineol + *P.acne* | 54.8 |
| Z | 0.2% Silver Oxide + *P.acne* | 494.3 |
| 17 | Andrographolide + 0.2% Silver Oxide + *P.acne* | 215.36 |

From the above table it is clear the selected antimicrobials synergistically interact with Andrographolide to provide benefit against P.acne (Example 14, 15 and 17). The effect is much more pronounced when some of the antimicrobials act together in combination with Andrographolide (Example 16).

## Claims

1. A topical composition comprising
(i) a compound of the Formula 1, andrographolide; 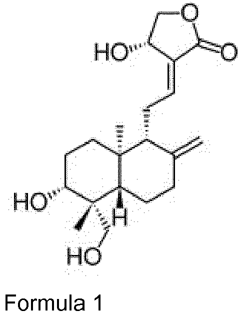
(ii) an antimicrobial agent; and
(iii) a cosmetically acceptable base;
wherein said antimicrobial agent is a combination of thymol and terpineol.

2. A composition as claimed in any one of the preceding claims wherein said composition is in the form of a cream, lotion, face wash, gel, emulsion, dentifrice, mouthwash, shampoo, hair oil or conditioner.

3. A composition as claimed in any one of the preceding claims comprising 0.001 to 5% compound of Formula 1

4. A composition as claimed in any one of the preceding claims comprising 0.0001 to 5% antimicrobial agent.

5. A composition as claimed in any one of the preceding claims wherein said compound of formula 1 is included as an extract of *Andrographis paniculata.*

6. A composition as claimed in any one of the preceding claims comprising 1 to 80% surfactant.

7. A composition as claimed in any one of the preceding claims for the treatment of acne.

8. A composition as claimed in any one of claims 1 to 6 for use in a therapeutic method of cleansing the external surface of the body comprising the steps of
(i) applying a composition as claimed in any one of the preceding claims on to the desired external surface; and optionally,
(ii) at least partially removing the composition from the surface.

9. A non-therapeutic method of cleansing the external surface of the body comprising the steps of
(i) applying a composition as claimed in any one of claims 1 to 6 on to the desired external surface; and optionally,
(ii) at least partially removing the composition from the surface.

## Patentansprüche

1. Topische Zusammensetzung, umfassend
(i) eine Verbindung der Formel 1, Andrographolid, 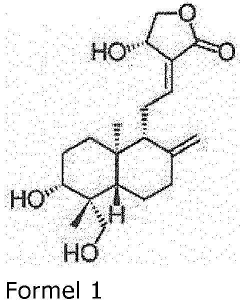
(ii) ein antimikrobielles Mittel und
(iii) eine kosmetisch akzeptable Grundlage,
wobei das antimikrobielle Mittel eine Kombination von Thymol und Terpineol ist.

2. Zusammensetzung, wie im Anspruch 1 beansprucht, wobei die Zusammensetzung in Form einer Creme, einer Lotion, eines Waschmittels, eines Gels, einer Emulsion, eines Zahnputzmittels, eines Mundwassers, eines Shampoos, eines Haaröls oder eines Konditioniermittels vorliegt.

3. Zusammensetzung, wie in irgendeinem der vorhergehenden Ansprüche beansprucht, umfassend 0,001 bis 5% der Verbindung der Formel 1.

4. Zusammensetzung, wie in irgendeinem der vorhergehenden Ansprüche beansprucht, umfassend 0,0001 bis 5% antimikrobielles Mittel.

5. Zusammensetzung, wie in irgendeinem der vorhergehenden Ansprüche beansprucht, wobei die Verbindung der Formel 1 als Extrakt von Andrographis paniculata eingeschlossen ist.

6. Verbindung, wie in irgendeinem der vorhergehenden Ansprüche beansprucht, umfassend 1 bis 80% Tensid.

7. Zusammensetzung, wie in irgendeinem der vorhergehenden Ansprüche beansprucht, zur Behandlung von Akne.

8. Zusammensetzung, wie in irgendeinem der Ansprüche 1 bis 6 beansprucht, zur Verwendung in einem therapeutischen Verfahren zum Reinigen der externen Oberfläche des Körpers, umfassend die Schritte
(i) Auftragen einer Zusammensetzung, wie in irgendeinem der vorhergehenden Ansprüche beansprucht, auf die gewünschte externe Oberfläche und optional
(ii) mindestens partielles Entfernen der Zusammensetzung von der Oberfläche.

9. Nicht-therapeutisches Verfahren zum Reinigen der externen Oberfläche des Körpers, umfassend die Schritte
(i) Auftragen einer Zusammensetzung, wie in irgendeinem der Ansprüche 1 bis 6 beansprucht, auf die gewünschte externe Oberfläche und optional
(ii) mindestens partielles Entfernen der Zusammensetzung von der Oberfläche.

## Revendications

1. Composition topique comprenant
(i) un composé de la Formule 1, andrographolide ; 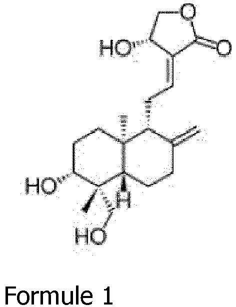
(ii) un agent antimicrobien ; et
(iii) une base cosmétiquement acceptable ;
dans laquelle ledit agent antimicrobien est une combinaison de thymol et terpinéol.

2. Composition selon l'une quelconque des revendications précédentes, dans laquelle ladite composition est dans la forme d'une crème, lotion, nettoyant visage, gel, émulsion, dentifrice, eau de bouche, shampoing, huile pour cheveux ou après-shampoing.

3. Composition selon l'une quelconque des revendications précédentes comprenant de 0,001 à 5 % de composé de Formule (1).

4. Composition selon l'une quelconque des revendications précédentes comprenant de 0,0001 à 5 % d'agent antimicrobien.

5. Composition selon l'une quelconque des revendications précédentes dans laquelle ledit composé de Formule 1 est inclus comme un extrait de *Andrographis paniculata.*

6. Composition selon l'une quelconque des revendications précédentes comprenant de 1 à 80 % de tensioactif.

7. Composition selon l'une quelconque des revendications précédentes pour le traitement de l'acné.

8. Composition selon l'une quelconque des revendications 1 à 6 pour une utilisation dans un procédé thérapeutique de nettoyage de la surface externe du corps comprenant les étapes de
(i) application d'une composition selon l'une quelconque des revendications précédentes sur la surface externe souhaitée ; et éventuellement,
(ii) élimination au moins partielle de la composition de la surface.

9. Procédé non-thérapeutique de nettoyage de la surface externe du corps comprenant les étapes de
(i) application d'une composition selon l'une quelconque des revendications 1 à 6 sur la surface externe souhaitée ; et éventuellement,
(ii) élimination au moins partielle de la composition de la surface.
